## Europäisches Patentamt

## European Patent Office

## Office européen des brevets

(19)

(11) Veröffentlichungsnummer: **0 038 317**
**B1**

(12)

# EUROPÄISCHE PATENTSCHRIFT

(45) Veröffentlichungstag der Patentschrift:
**14.11.84**

(21) Anmeldenummer: **81890050.8**

(22) Anmeldetag: **20.03.81**

(51) Int. Cl.³: **C 07 D 307/50,** D 21 C 11/00,
B 01 D 3/34

(54) Verfahren zur Gewinnung von Chemikalien aus sauren Hydrolysaten von Pflanzen.

(30) Priorität: **10.04.80 AT 1931/80**

(43) Veröffentlichungstag der Anmeldung:
**21.10.81 Patentblatt 81/42**

(45) Bekanntmachung des Hinweises auf die Patenterteilung:
**14.11.84 Patentblatt 84/46**

(84) Benannte Vertragsstaaten:
**BE CH DE FR GB IT LI NL SE**

(56) Entgegenhaltungen:
**AT - A - 356 509
DE - A - 2 423 272
DE - B - 1 029 661
FR - A - 1 510 864
US - A - 2 140 694**

**J.M. COULSON et al.: "Chemical Engineering", Band 3,
Kapitel 1, 1979, Seiten 1-104, Pergamon Press Oxford,
G.B. J.C. LEE: "Reactor design- general principles"
TAPPI JOURNAL OF THE TECHNICAL ASSOCIATION
OF THE PULP AND PAPER INDUSTRY, Band 52, Nr. 3,
März 1969, Seiten 486-490 Atlanta, G.A., U.S.A. L.L.
ZOCH et al.: "Furfural from spent sodium-base acid
sulfite pulping liquor"
CHEMIE-INGENIEUR-TECHNIK, Band 45, Nr. 14, Juli
1973, Seiten 942-945, Verlag Chemie, Weinheim, DE. B.**

(73) Patentinhaber: **VEREINIGTE EDELSTAHLWERKE
AKTIENGESELLSCHAFT (VEW), Elisabethstrasse 12,
A-1010 Wien (AT)**

(72) Erfinder: **Kanzler, Walter, Dipl.-Ing.,
Viktor-Geramb-Weg 13, A-8010 Graz (AT)**
Erfinder: **Schedler, Johannes, Dipl.-Ing.,
Rupertistrasse 119, A-8042 Graz - St. Peter (AT)**

(74) Vertreter: **Widtmann, Georg, Dr., Elisabethstrasse 12,
A-1010 Wien (AT)**

(56) Entgegenhaltungen: (Fortsetzung)
**HEGNER et al.: "Möglichkeiten der Berechnung bei
heteroazeotroper Destillation"
Chem. Ing. Techn. 38 (1966) S. 1053-59**

## Beschreibung

Die Erfindung bezieht sich auf ein Verfahren zur Gewinnung von Furfurol, Essigsäure und Ameisensäure und anderen organischen Verbindungen aus sauren Hydrolysaten von Pflanzen.

Furfurol kann durch Hydrolyse von pentosanhaltigen Pflanzen oder deren Abfällen gewonnen werden. Dabei wird das Pflanzenmaterial bei Temperaturen unter 200 °C in einem Hydrolyseapparat (Kocher) unter sauren Bedingungen behandelt. Das Furfurol wird daraufhin durch Behandlung mit Wasserdampf (ca. 20 t Dampf/t Furfurol) gewonnen. Dieses Verfahren ist sehr energieaufwendig und es wird daher in Furfurolanlagen meist das ausgekochte Pflanzenmaterial nach der Hydrolyse verbrannt und die Verbrennungswärme zur Erzeugung des nötigen Wasserdampfes verwendet.

Bei Zellstoffabriken steht die Gewinnung von Zellstoff an erster Stelle und es werden im allgemeinen die Pentosane zusammen mit den Lignin- und sonstigen Hydrolyseprodukten in der Ablauge eingedampft und verbrannt. Insbesondere beim Aufschluss von Laubholz und Einjahrespflanzen werden dabei grosse Mengen von Pentosanen (ca. 20% des Rohstoffes) verbrannt. Es wurde daher immer wieder versucht, Furfurol aus Sulfitablaugen durch Umwandlung der Pentosane zu gewinnen, z.B. durch mehrstündige Druckerhitzung der Sulfitablauge bei 150 bis 180 °C und anschliessende Destillation der Sulfitablauge. Infolge der langen Erhitzung der Sulfitablauge wurde ein grosser Teil des erzeugten Furfurols wieder zersetzt. Weiters erforderte die Destillation des Furfurols aus der Sulfitablauge grosse Mengen von Dampf, beispielsweise 50 bis 60 t Dampf pro t Furfurol.

Aus der DE-B-1 029 661 ist ein Verfahren zur Umwandlung von Pentosanen zu Furfurol bekannt, bei dem Zellstoffablauge, welche vorher teilweise eingeengt worden ist, durch einen Wärmetauscher geführt und danach in einen Überhitzer eingebracht wird, in welchem der überwiegende Teil der Reaktion zu Furfurol erfolgt, wonach die so erhitzte Ablauge als Heizmedium durch den vorher erwähnten Wärmetauscher geleitet wird. Danach erfolgen weitere Einengungen der Ablaugen und destillative Abtrennung des Furfurols. Die bei diesem Verfahren zwingend erforderliche Voreindickung der Lauge erfolgt bei Temperaturen bis 110 °C, wobei in geringem Mass schon die Pentosen-zu-Furfurol-Umwandlung stattfindet. Die gemäss DE-B-1 029 661 voreingedickte Lauge neigt infolge ihrer höheren Konzentration und des erhöhten Furfurolgehaltes zu Verharzungen, was insbesondere im Umsetzungs-Überhitzer und danach im Ablaugen-Vorwärm-Wärmetauscher zu Problemen infolge oft spontaner Verkrustungen führen kann.

Ergänzend sei erwähnt, dass in Chem. Eng. 3, Kap. 1, (1979), S. 8, 9 allgemein eine Vorrichtung beschrieben ist, die aus der oben genannten Kombination von Reaktor und von diesem aus mit dem Reaktionsmedium als Heizmedium versorgtem Gegenstromwärmetauscher gebildet ist.

In der FR-A-1 510 864 ist ein Ablauge-Konzentrier-Verfahren beschrieben, bei dem ebenfalls eine voreingedickte Ablauge in einem einfachen Reaktor mit 150–300 °C eingebracht wird, wo die Umwandlung der Pentosane zu Furfurol stattfindet, wonach eine destillative Aufarbeitung erfolgt. Infolge der wesentlich höheren Reaktionstemperatur ist die obengenannte Verkrustungsgefahr als Folge der auch hier höheren Laugenkonzentration erhöht.

Ergänzend sei angeführt, dass gemäss Tappi, I. Techn. Ass. of the Pulp and Paper Ind. 52, Nr. 3 (1969), S. 487 eine Umwandlung der Pentosane zu Furfurol bei 240–260 °C innerhalb von etwa 10 s erfolgen kann.

Was die Aufarbeitung von Furfurol enthaltenden Brüden bzw. deren Kondensaten betrifft, so ist in der AT-A-356 509 ein Verfahren beschrieben, bei dem durch Extraktion mit flüssigen Ionenaustauschern die Inhaltsstoffe wirtschaftlich gewonnen werden können. Der Nachteil dieses Verfahrens besteht darin, dass zwar die gesamte Essigsäure gewonnen werden kann, die beim Aufschluss entsteht, jedoch die Pentosane in Form von Xylose werden weiterhin verbrannt.

Die vorliegende Erfindung hat sich zur Aufgabe gestellt, ein Verfahren zu schaffen, bei dem ein grosser Teil der in den Hydrolysaten (z.B. Sulfitablaugen) enthaltenen Pentosane und aus ihnen Furfurol gewonnen werden kann, wobei die Pentosane ohne grosse Verluste durch Zersetzung rasch in Furfurol umgewandelt werden und das erzeugte Furfurol danach energiesparend gewonnen wird.

Das erfindungsgemässe Verfahren zur Gewinnung von im wesentlichen Furfurol, Ameisensäure und Essigsäure aus sauren Hydrolysaten von Pflanzen, insbesondere Sulfitablaugen, bei welchen die Umwandlung der enthaltenen Pentosen zu Furfurol, vorzugsweise mit Ansäuerung, durch Erhitzen in einem Reaktor erfolgt und das im Reaktor zusätzlich erhitzte und umgesetzte Hydrolysat als Heizmedium in einem Gegenstromwärmetauscher umzusetzendes Hydrolysat vorerwärmt und Furfurol als Destillat gewonnen wird, ist dadurch gekennzeichnet, dass dem Reaktor, vorzugsweise Rohrreaktor, unumgesetztes, einer vorherigen Einengung nicht unterworfenes Hydrolysat, vorzugsweise Sulfitablauge, zugeführt und dort auf Temperaturen von über 200 °C, vorzugsweise von 220 bis 300 °C, erhitzt wird, und dass nach Führung des dann umgesetzten Hydrolysates als Heizmedium durch den Gegenstromwärmeaustauscher Furfurol gemeinsam mit im wesentlichen Ameisensäure und Essigsäure als Destillat gewonnen wird, und gegebenenfalls dieses Destillat mit einem Extraktionsmittel mit einer 10 bis 80%igen Lösung von Phosphinoxiden, vorzugsweise Trioctylphosphinoxid, in einem aliphatischen Kohlenwasserstoff mit einem Siedepunkt zwischen 150 und 220 °C extrahiert wird.

Die im nicht voreingeengten Hydrolysat enthaltene Xylose wird erst im Reaktor in Furfurol umgewandelt. Das den Reaktor verlassende, umgesetzte Hydrolysat gibt im Gegenstromwärmetauscher seine Wärme an das unbehandelte, unumgesetzte Hydrolysat ab, und heizt es bis auf wenige Grade unter die Umwandlungstemperatur auf. Die noch nötige zusätzliche Wärmemenge wird dann dem Reaktor durch eine Mantelheizung oder durch innenliegende Heizregister zugeführt. Das so behandelte, Furfurol enthaltende Hydrolysat wird danach einer, vorzugsweise mehrstufigen, Eindampfanlage zugeführt, in der das Hydrolysat auf eine Konzentration von ca. 50% eingedickt und im Anschluss daran verbrannt werden kann. Die bei der Eindampfung ausgedampften Brüden bzw. deren in jeweils vorhergehenden Eindampfstufen anfallenden Kondensate enthalten das im umgewandelten Hydrolysat vorliegende Furfurol und die flüchtigen Säuren fast vollständig.

Die Geschwindigkeit der Umwandlung von Xylose in Furfurol im Reaktor bei Temperaturen zwischen 200 und 300 °C ist hoch; trotz dieser hohen Temperaturen sind überraschenderweise Zersetzungsreaktionen und damit Probleme mit Verharzung und Verkrustung unerwartet gering. Dies dürfte eine Folge des Einsatzes der nicht voreingeengten Hydrolysate sein.

Die gegebenenfalls vorgesehene Art der Aufarbeitung durch Extraktion ist besonders günstig, da bei der erfindungsgemässen Umwandlung zu Furfurol in nicht voreingedickten Hydrolysaten grössere Wassermengen anfallen und das Furfurol in den Kondensaten verdünnter vorliegt.

Bei diesen Temperaturen von über 200 °C ist eine Verweilzeit des Hydrolysates von weniger als 10 min, insbesondere weniger als 1 min im Reaktor günstig, was eine hohe Ausbeute bei gleichzeitiger Vermeidung der oben erwähnten Verharzungsgefahr sichert. Die im Reaktor erfolgende Erhitzung der gesamten Hydrolysatmenge auf die genannten Temperaturen hat neben der problemlosen Umwandlung zu Furfurol den weiteren Vorteil, dass für die nachfolgende Eindickstufen für die Ablauge deren Wärmeinhalt ausreicht und Zusatzenergie überflüssig ist.

Eine besonders einfache Verfahrensvariante, wobei gleichzeitig die anfallenden organischen Säuren verwendet werden können, besteht darin, dass dem Hydrolysat vor Einbringung in den Reaktor ein Teil der aus dem Destillat gewonnenen Ameisensäure und/oder Essigsäure zugeführt wird. Dadurch wird eine Fremdsäureeinbringung vermieden. Bei der gegebenenfalls vorgesehenen Aufarbeitung durch Extraktion kann das erhaltene, beladene Extraktionsmittel besonders einfach aufgearbeitet werden. Hierbei wird das Extraktionsmittelgemisch durch Destillation regeneriert wobei der überwiegende Teil des im beladenen Extraktionsmittel von der Extraktion her noch vorhandenen Wassers abdestilliert und, worauf gegebenenfall bei Unterdruck eine Mischung, vorzugsweise ein Azeotrop, bestehend aus Furfurol, den Säuren, Verunreinigungen und Lösungsmittel abdestilliert. Bei der Kondensation dieser Mischung der Kopfprodukte bilden sich mindestens zwei Phasen, wobei die eine aus dem Verdünnungsmittel und die andere aus den Produkten und Verunreinigungen besteht, wobei sich gegebenenfalls bereits Furfurol als eigene Phase abscheiden kann.

Insbesondere bei einem niedrigen Ameisensäuregehalt wird gemäss der vorliegenden Erfindung aus der bei der Destillation des Extraktionsmittels erhaltenen Produktmischung ein Gemisch, vorzugsweise Azeotrop aus Verunreinigungen, Wasser, Ameisensäure und Essigsäure, sowie Spuren von Furfurol abdestilliert, wobei gegebenenfalls dieses Gemisch dem Hydrolysat vor dessen Einbringung in den Reaktor zugeführt wird und anschliessend das im Sumpf verbleibende Gemisch mit Essigsäure und Furfurol destillativ aufgetrennt wird.

Als besonders vorteilhaft hat es sich erwiesen, wenn aus der bei der Destillation des Extraktionsmittels erhaltenen Produktmischung ein Gemisch, vorzugsweise Azeotrop, mit Verunreinigungen, Wasser, Spuren von Ameisensäure, Essigsäure und Furfurol abdestilliert wird, worauf diesem ein Schleppmittel für das Wasser, vorzugsweise Di-n-Propyläther oder Äthyl-n-Butyläther, zugesetzt wird und das Wasser gemeinsam mit dem Schleppmittel abdestilliert wird.

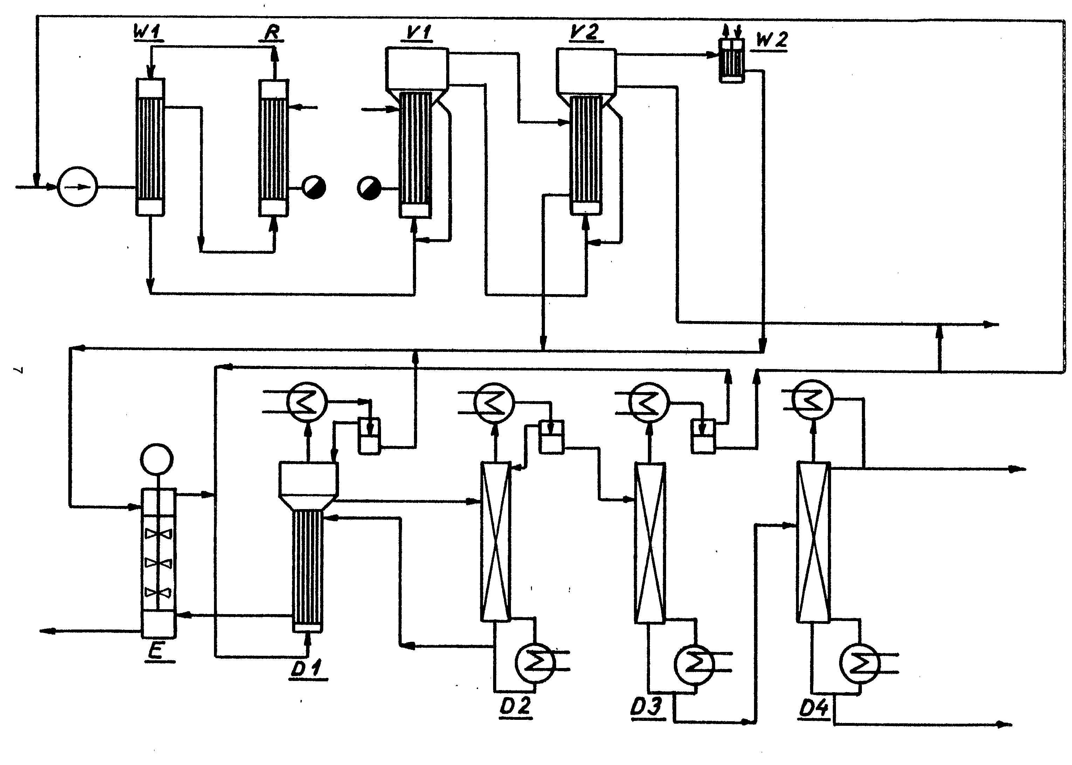

Im folgenden wird die Erfindung an Hand eines Beispieles und der Zeichnung, die ein Fliessschema zeigt, näher erläutert.

Beispiel

Eine Sulfitablauge einer Magnesiumbisulfitkochung von Laubhölzern hat einen pH-Wert von 1,6 und enthält u.a. 50 g/l Xylose, 17 g/l Essigsäure und 0,6 g/l Ameisensäure. Diese Sulfitablauge wird mit einem Säuregemisch angesäuert, mit einer Pumpe auf einen Druck von 80 bar gebracht und im Gegenstromwärmeaustauscher (W1) auf 270 °C erhitzt. Die Strömungsmenge beträgt 125 m³/h. Die erwärmte Sulfitablauge strömt mit einer Geschwindigkeit von 1 m/s durch den Reaktor R und wird dabei auf 280 °C angewärmt. Bei einer Verweilzeit von ca. 20 s werden 20 g/l Furfurol gebildet. Diese Sulfitablauge aus dem Reaktor strömt wieder durch den Gegenstromwärmetauscher W1 und gibt dort ihre Wärme an die frische Sulfitablauge ab. In der mehrstufigen Eindampfanlage (V1 und V2) wird die Ablauge von ca. 10 Gew.-% auf ca. 50 Gew.-% Trockensubstanz eingedampft und danach einer Verbrennung zugeführt. Im Wärmetauscher W2 werden die Brüden kondensiert und dem Extraktor E zugeführt, in welchem 100 m³/h Brüdenkondensat mit einem Gehalt von ca. 20 g/l Furfurol, 17 g/l Essigsäure und 0,6 g/l Ameisensäure in einer Stunde mit 100 m³ einer 30gewichtsprozentigen Lösung von Trioctylphosphinoxid in Undekan im Gegenstrom in Kontakt gebracht werden. Aus dem beladenen Extraktionsmittel wird im Verdampfer D1 eine Mischung (Azeotrop) aus Undekan und Wasser abdestilliert. Nach der Kondensation und Phasenabscheidung wird das Undekan in den Verdampfer zurückgeführt. Das beladene Extraktionsmittel, von dem nun der Grossteil des Wassers entfernt ist, wird in der Destillationskolonne D2 regeneriert und nach Wärmeabgabe im Verdampfer D1 wieder dem Extraktor E zugeführt.

Am Kopf der Kolonne D2 wird ein Gemisch aus Undekan, Furfurol, Essigsäure, Ameisensäure, Wasser und Verunreinigungen kondensiert und die Phasen werden in einem Abscheider W2 getrennt, wobei das Undekan der Kolonne D2 als Rücklauf aufgegeben wird.

Da Produktgemisch wird daraufhin in der Kolonne D3 einer weiteren Destillation unterworfen, wobei ein Gemisch (Azeotrop) aus dem Wasser, der Ameisensäure, einem kleinen Teil der Essigsäure und den Verunreinigungen ausgetrieben wird. Das Produktgemisch ist nun wasserfrei und besteht nur mehr aus Essigsäure und Furfurol, welche in der Kolonne D4 destillativ getrennt werden können. Insgesamt werden aus 125 m³ Sulfitablauge 2000 kg Furfurol und 1800 kg Essigsäure gewonnen. Das in der Kolonne D3 ausgetriebene Wasser-Säuregemisch kann zum Ansäuern der Sulfitablauge verwendet werden.

## Patentansprüche

1. Verfahren zur Gewinnung von im wesentlichen Furfurol, Ameisensäure, Essigsäure und anderen organischen Verbindungen aus sauren Hydrolysaten von Pflanzen, insbesondere Sulfitablaugen, bei welchen die Umwandlung der enthaltenen Pentosen zu Furfurol, vorzugsweise mit Ansäuerung durch Erhitzen in einem Reaktor erfolgt und das im Reaktor zusätzlich erhitzte und umgesetzte Hydrolysat als Heizmedium in einem Gegenstromwärmetauscher umzusetzendes Hydrolysat vorerwärmt und Furfurol als Destillat gewonnen wird, dadurch gekennzeichnet, dass dem Reaktor, vorzugsweise Rohrreaktor, unumgesetztes, einer vorherigen Einengung nicht unterworfenes Hydrolysat, vorzugsweise Sulfitablauge, zugeführt und dort auf Temperaturen von über 200°C, vorzugsweise von 220 bis 300°C, erhitzt wird, und dass nach Führung des dann umgesetzten Hydrolysates als Heizmedium durch den Gegenstromwärmeaustauscher Furfurol gemeinsam mit im wesentlichen Ameisensäure und Essigsäure als Destillat gewonnen wird, und gegebenenfalls dieses Destillat mit einem Extraktionsmittel mit einer 10 bis 80gewichtsprozentigen Lösung von Phosphinoxiden, vorzugsweise Trioctylphosphinoxid, in einem aliphatischen Kohlenwasserstoff mit einem Siedepunkt zwischen 150 und 220°C extrahiert wird.

2. Verfahren nach Anspruch 1, dadurch gekennzeichnet, dass die Verweilzeit des Hydrolysates im Reaktor auf kleiner als 10 min, vorzugsweise kleiner als 1 min, eingestellt wird.

3. Verfahren nach Anspruch 1 oder 2, dadurch gekennzeichnet, dass dem Hydrolysat vor Einbringung in den Reaktor ein Teil der aus dem Destillat bei der weiteren Aufarbeitung gewonnenen Ameisensäure und/oder Essigsäure zugeführt wird.

4. Verfahren nach einem der Ansprüche 1 bis 3, dadurch gekennzeichnet, dass zur Aufarbeitung des beladenen Extraktionsmittelgemisches dieses durch Destillation regeneriert wird, wobei vorzugsweise der überwiegende Teil des im beladenen Extraktionsmittel gelösten Wassers abdestilliert wird, worauf, gegebenenfalls bei Unterdruck, eine Mischung, vorzugsweise ein Azeotrop, bestehend aus Furfurol, Säuren, Verunreinigungen und Lösungsmittel abdestilliert wird.

5. Verfahren nach Anspruch 1 oder 4, dadurch gekennzeichnet, dass aus der bei der Destillation des Extraktionsmittels erhaltenen Produktmischung ein Gemisch, vorzugsweise Azeotrop, aus Verunreinigungen, Wasser, Ameisensäure und Essigsäure, sowie Spuren von Furfurol abdestilliert wird, wobei gegebenenfalls dieses Gemisch dem Hydrolysat vor dessen Einbringung in den Reaktor zugeführt wird und anschliessend das im Sumpf verbleibende Gemisch mit Essigsäure und Furfurol destillativ aufgetrennt wird.

6. Verfahren nach Anspruch 1, dadurch gekennzeichnet, dass aus der bei der Destillation des Extraktionsmittels erhaltenen Produktmischung ein Gemisch, vorzugsweise Azeotrop, mit Verunreinigungen, Wasser, Spuren von Ameisensäure, Essigsäure und Furfurol abdestilliert wird, worauf diesem ein Schleppmittel für das Wasser, vorzugsweise Di-n-propyläther und/oder Äthyl-n-butyläther zugesetzt wird und das Wasser gemeinsam mit dem Schleppmittel abdestilliert wird.

## Claims

1. A method for extracting essentially furfurol, formic acid, acetic acid and other organic compounds from acid hydrolysates of plants, in particular spent sulphite lyes, in which the conversion to furfurol of the pentoses contained is performed by heating in a reactor, preferably with acidification, and the hydrolysate additionally heated and converted in the reactor, as the heating medium, preheats the hydrolysate to be converted in a counterflow heat exchanger and furfurol is obtained as the distillate, characterized in that a hydrolysate, preferably spent sulphite lye, which has not previously been concentrated, is fed to the reactor, preferably a tube reactor, and is heated there to temperatures of above 200°C, preferably from 220 to 300°C, and, after the subsequently converted hydrolysate has been led as the heating medium through the counterflow heat exchanger, furfurol is obtained together with essentially formic acid and acetic acid as the distillate, and if necessary this distillate is extracted by an extracting agent comprising a solution of 10 to 80% by weight phosphine oxides, preferably trioctyl phosphine oxide, in an aliphatic hydrocarbon with a boiling temperature of between 150 and 220°C.

2. A method according to claim 1, characterized in that the period of time during which the hydrolysate remains in the reactor is set at less than 10 minutes, preferably less than 1 minute.

3. A method according to claim 1 or 2, characterized in that some of the formic acid und/or acetic acid extracted from the distillate in the further recovery process is delivered to the hydrolysate before it is fed into the reactor.

4. A method according to one of claims 1 to 3, characterized in that for the recovery of the charged extracting agent mixture the latter is regenerated by distillation, the greater part of the water dissolved in the charged extracting agent preferably being distilled off, whereupon a mixture, preferably an azeotrope, consisting of furfurol, acids impurities and solvents is distilled off, if necessary under low pressure.

5. A method according to claim 1 or 4, characterized in that a mixture, preferably an azeotrope, of impurities, water, formic acid and acetic acid as well as traces of furfurol is distilled off from the product mixture obtained when the extracting agent is distilled, this mixture if necessary being delivered to the hydrolysate before the latter is introduced into the reactor and subsequently the mixture remaining in the sump being separated by distillation with acetic acid and furfurol.

6. A method according to claim 1, characterized in that a mixture, preferably an azeotrope, with impurities, water, traces of formic acid, acetic acid and furfurol is distilled off from the product mixture obtained during the distillation of the extracting agent, whereupon an entrainer for the water, preferably di-n-propylether and/or ehtyl-n-butylether, is added to the mixture, and the water, together with the entrainer, is distilled off.

**Revendications**

1. Procédé de préparation, essentiellement de furfural, d'acide formique, d'acide acétique et d'autres substances organiques, à partir d'hydrolysats acides de plantes, en particulier de lessives de sulfite résiduaires, où la conversion des pentoses contenus en furfural s'effectue de préférence avec une acidification, par chauffage dans un réacteur, l'hydrolysat ayant subi un chauffage supplémentaire dans le réacteur et ayant réagi assurant, en tant que fluide caloporteur, le préchauffage de l'hydrolysat qui va réagir dans un échangeur de chaleur à contre-courant, le furfural étant obtenu sous forme de distillat, caractérisé en ce que l'on amène au réacteur, de préférence un réacteur tubulaire, un hydrolysat n'ayant pas réagi, qui n'a pas été soumis à une concentration préalable, de préférence une lessive de sulfite résiduaire, et qu'on l'y chauffe à des températures supérieures à 200 °C, de préférence de 220 à 300 °C, et que, après envoi de l'hydrolysat, qui a maintenant réagi, en tant que fluide caloporteur à travers l'échangeur de chaleur à contre-courant, on obtient du furfural en même temps qu'essentiellement de l'acide formique et de l'acide acétique sous forme d'un distillat, ce distillat étant éventuellement extrait à l'aide d'un agent d'extraction, avec und solution à 10 à 80% en poids d'oxydes de phosphine, de préférence d'oxyde de trioctylphosphine, dans un hydrocarbure aliphatique ayant un point d'ébullition compris entre 150 et 220 °C.

2. Procédé selon la revendication 1, caractérisé en ce que le temps de séjour de l'hydrolysat dans le réacteur est ajusté à moins de 10 min, de préférence à moins de 1 min.

3. Procédé selon l'une des revendications 1 ou 2, caractérisé en ce que l'on introduit dans l'hydrolysat, avant introduction dans le réacteur, une partie de l'acide formique et/ou de l'acide acétique extrait du distillat lors du traitement ultérieur.

4. Procédé selon l'une des revendications 1 à 3, caractérisé en ce que, pour le traitement du mélange chargé d'agents d'extraction, ce dernier est régénéré par distillation, ce à l'occasion de quoi la partie principale de l'eau dissoute dans l'agent d'extraction chargé est chassée par distillation, ce après quoi, éventuellement en dépression, on chasse par distillation un mélange, de préférence azéotrope, constitué de furfural, d'acides, d'impuretés et de solvant.

5. Procédé selon la revendication 1 ou 4, caractérisé en ce qu'on chasse par distillation, du mélange de produits obtenu lors de la distillation de l'agent d'extraction, un mélange, de préférence azéotrope, d'impuretés, d'eau, d'acide formique et d'acide acétique, ainsi que de traces de furfural, ce à l'occasion de quoi ce mélange est éventuellement envoyé à l'hydrolysat avant son introduction dans le réacteur, le mélange restant dans le fond, avec de l'acide acétique et du furfural, étant ensuite séparé par distillation.

6. Procédé selon la revendication 1, caractérisé en ce qu'on chasse par distillation, du mélange de produits obtenu lors de la distillation de l'agent d'extraction, un mélange, de préférence azéotrope, contenant des impuretés, de l'eau, des traces d'acide formique, de l'acide acétique et du furfural, ce après quoi on ajoute à ce dernier un agent d'entraînement pour l'eau, de préférence le di-n-propyléther et/ou l'éthyl-n-butyléther, l'eau étant chassée par distillation en même temps que l'agent d'entraînement.